# EUROPEAN PATENT APPLICATION

(11) **EP 1 447 109 A2**
(43) Date of publication of application: **18.08.2004**
(21) Application number: 04250756.6
(22) Date of filing: 12.02.2004
(51) Int. Cl.: A61M 5/32

(54) **Medical waste disposal containers**

(30) Priority: 12.02.2003 GB 0303218
(71) Applicant: Daniels Healthcare Ltd., Tring, Hertordshire HP23 6AF (GB)
(72) Inventor: Hatton, William Maurice c/o Daniels Healthc.Ltd., Kidlington Oxon OX5 1JD (GB)
(74) Representative: Baldwin, Mark

(57) **Abstract**

A medical waste disposal container (10) comprises a hollow container portion (12) and a top cover (24) securable to an upper end region (20) of the container portion by a snap-fitting engagement arrangement. The upper end region (20) is provided with an opening (22) through which waste is received, the top cover includes an inlet (30) through which waste can be inserted for passage to the opening (22) and is provided with a first portion (20) of the snap-fitting engagement arrangement. The container portion (12) is provided with a second portion of the snap-fitting engagement arrangement which is arranged such that when the top cover and upper end region of the container portion are brought together, the first and second portions snap-fittingly engage to secure the top cover in a position covering the opening (22). The waste disposal container is further provided with a guide arrangement (156) for guiding the top cover towards the position covering the opening (22). The guide arrangement comprises guide members (156) provided on at least one of the top cover and container portion for engaging the other of the top cover and container portion as they are brought together for guiding the top cover to the position covering the opening (22).

## Description

The invention relates to medical waste disposal containers, which are typically used for the disposal of so-called sharps, such as, the needles used on hypodermic syringes.

Medical waste disposal containers, often referred to as sharps bins, typically comprise two major components; a hollow container portion in which the waste is stored and a lid for the container. It is preferable from the point of view of transportation costs that such sharps bins are shipped to the end user in a disassembled condition for assembly at the point of use. This allows the parts to be stacked one inside another, thereby reducing the transportation volume and the amount of packaging required.

It is known to provide sharps bins that comprise a hollow container portion having an upper end that defines an opening through which waste is received and a top cover positioned over the opening. The top cover has an inlet through which used medical waste and in particular needles or hypodermic syringes, with or without their needles, can be inserted into the sharps bin. The opening of the top cover is provided with a lid or other form of closure to permit the sharps bin to be securely closed once full. One example of such a sharps container is the 4 litre Sharpsafe™ produced by Frontier Plastics Limited.

These known containers are typically essentially rectangular in plan. The perimeter of the upper end of the container portion is usually configured to snap-fit in a channel that is defined by the lid and as a result of the shape of the container portion, the channel will also be substantially rectangular. One result of this is that when the user fits the top cover to the container portion, he or she must press down on all four comers of the top cover to ensure that it snap-fits around the entire upper end of the container portion. In practice it has been found that all four sides of the top cover are often not properly fitted on the container portion, with the result that these sharps bins are often used with the top cover not securely fitted to the container portion. This is of course highly undesirable and potentially dangerous.

Sharps bins are typically made of a thermoplastics material. Another problem that can arise with such sharps bins is that where the container portion has relatively long sidewalls, these may bow or flex making it difficult to mate the upper end of the container portion with the channel provided in the top cover. This is a particular problem where snap-fit connections are to be made, since these will usually rely on the deflection of a part on one of the top cover or container portion by a part carried on the other. If the parts do not mate due to such flexing or bowing such deflection cannot take place and, thus, the top cover and container portion will not be properly secured. Again, this is highly undesirable and potentially dangerous.

In view of these problems, some manufacturers of sharps bins elect to assemble the top cover to the container portion as a part of the manufacturing process. This may be by hand, but in many cases a press is used to force the top cover onto the container portion. This prior assembly is undesirable since it adds to both the manufacturing and transportation cost.

An object of the invention is to at least in part alleviate one or more of the above-mentioned problems, or to provide an alternative choice of waste disposal container.

The invention provides a medical waste disposal container comprising a hollow container portion and a top cover securable to an upper end region of said container portion by a snap-fitting engagement arrangement, said upper end region being provided with an opening through which waste is received, said top cover including an inlet through which waste can be inserted for passage to said opening and being provided with a first portion of said snap-fitting engagement arrangement and said container portion being provided with a second portion of said snap-fitting engagement arrangement which is arranged such that when said top cover and upper end region of the container portion are brought together, said first and second portions snap-fittingly engage to secure said top cover in a position covering said opening, said waste disposal container being further provided with a guide arrangement for guiding said top cover towards said position, said guide arrangement comprising guide members provided on at least one of said top cover and container portion for engaging the other of said top cover and container portion as they are brought together for guiding said top cover to said position.

The invention also includes a waste disposal container means having an upper end provided with an opening through which waste is received and a top cover means securable to said container means by push-fitting so as to cover said opening, said top cover means having inlet means through which waste can be inserted to pass to said opening and guide means for engaging said container portion to guide said top cover means to a fully engaged position during said push-fitting.

The invention also includes a waste disposal container means having an upper end provided with an opening through which waste is received and top cover means securable to said container means, said top cover means comprising inlet means through which waste can be inserted to pass to said opening and internally disposed support means by means of which a plurality of such top cover means can be stacked in a nested condition. Preferably, there are push-fitting means by which said top cover means is securable to said container means.

The invention also includes a medical waste disposal container comprising a hollow container portion having an opening at an upper end region thereof for receiving waste and a top cover securable to said hollow container portion so as to cover said opening, said top cover including an inlet through which waste can be inserted for passage to said opening and an internal support arrangement for locating and supporting a second such top cover in a nested condition. Preferably, the support arrangement comprises a plurality of members provided on an inner surface of said top cover for engaging an outer surface of said second such top cover when in said nested condition. Preferably, said members provided on said inner surface of said top cover are arranged to engage said container portion to guide said top cover towards said position covering said opening when the top cover is brought towards said container portion to be secured thereto. Preferably, at least one said member is arranged such that subsequent to said engagement of the container portion, it can deflect a second portion of said snap-fitting engagement arrangement that is provided on said container portion to ensure said second portion snap-fittingly engages a first portion of said snap-fitting engagement arrangement that is provided on said top cover.

The invention also includes a wall mounting bracket for a medical waste disposal container, said bracket comprising a body portion that defines a support area for such a medical waste disposal container and a separate resilient locking device carried by said body portion and having a normal position in which it engages a said container mounted to said body portion to lock said container to the body portion, said resilience permitting movement of said locking device from said locking position to permit said container to be mounted to or removed from said body portion.

Preferably, said resilient locking device is securable to said body portion by push-fitting. Preferably, said body part has opposed inclined walls for cooperably engaging respective walls of a dovetail groove provided in said medical waste disposal container.

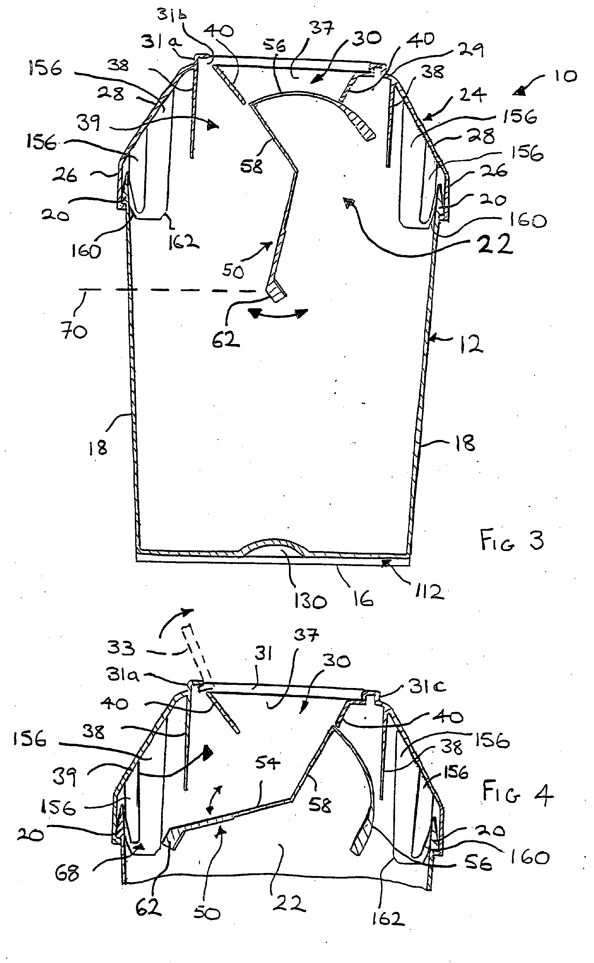

In this specification, the terms upper, lower and upwardly and downwardly have been used merely for convenience and ease of description and unless indicated otherwise, refer to the orientation of the waste disposal container shown in Figure 3.

In order that the invention may be well understood an embodiment thereof, which is given by way of example only, will now be described with reference to the drawings, in which:
Figure 1 is a perspective view of a medical waste disposal container with the door of its top cover removed;
Figure 2 is a perspective view of a door for the top cover of the waste disposal container of Figure 1;
Figure 3 is a transverse section midway along the length of the container showing a tumbler device of the waste disposal unit in a first position;
Figure 4 is a partial view corresponding to Figure 3 showing the tumbler device in a second position;
Figure 5 is an upside-down perspective view of the top cover of the medical waste disposal container with the tumbler device removed;
Figure 6 is a perspective view showing a second top cover stacked for transport with a top cover of Figure 5 with parts of the second top cover omitted purely for convenience of illustration;
Figure 7 is a perspective view of the tumbler device;
Figure 8 is an enlargement of a portion of Figure 3;
Figure 9 is an exploded perspective view of a wall mounting bracket for the medical waste disposal container; and
Figure 10 is a front elevation of the assembled wall mounted bracket.

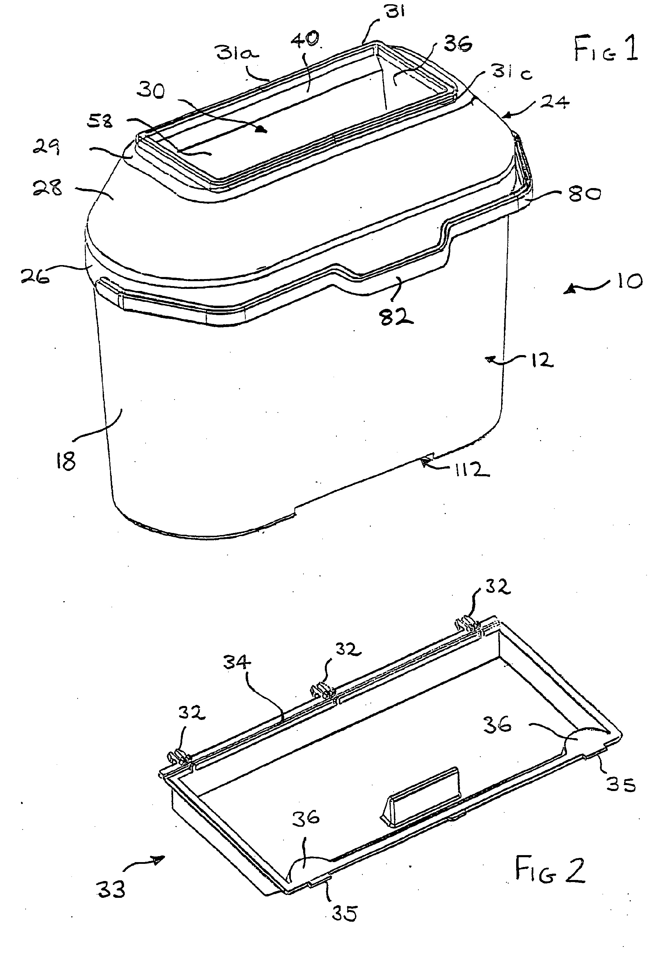

Referring primarily to Figures 1 to 4, a medical waste disposal container 10 comprises a hollow container portion 12. The container portion 12 comprises a bottom wall 16 and a peripheral wall 18 extending upwardly from the bottom wall to an upper end 20. The upper end 20 of the peripheral wall 18 defines an opening 22 through which waste is received. The peripheral wall 18 tapers outwardly from the bottom wall 16 towards its upper end 20. This allows container portions 12 to be nested for convenience of packing and transportation. Advantageously, lugs or projections (not shown) may be provided on an inner surface of the container portion 12 adjacent the lower end thereof to improve the stackablilty of the container portions.

The upper end 20 of the container portion 12 has a generally triangular cross-section and forms a barb-like part of a snap-fitting engagement arrangement by which a top cover 24 can be secured to the container portion. In plan the peripheral wall 18 can be seen as two semi-circular end wall portions having their respective ends interconnected by respective generally straight sidewall portions.

The top cover 24 is an integral unit comprising a first peripheral wall 26 that defines a lower end region of the top cover. The first peripheral wall 26 slopes inwardly in the upwards direction and in plan corresponds substantially to the plan of the upper end 20 of the container portion 12. A second peripheral wall 28 extends upwards from the first peripheral wall 26. The second peripheral wall 28 slopes inwardly in the upward direction and in plan corresponds substantially to the upper end 20 of the container portion 12; although with progressively smaller dimensions. The angle of inclination of the second peripheral wall 28 is greater than that of the first peripheral wall 26. As an alternative to the angled first peripheral wall 26, shown in the drawings, the wall may extend vertically or be angled in the opposite direction.

At its upper end 29, the second peripheral wall flattens to define an upper wall in which a rectangular inlet 30 is provided through which waste can be inserted for passage to the container portion opening 22. A perimeter wall 31 extends around the inlet 30 and is provided on one of its longer sides 31a with apertures 31b (Figure 3) for receiving connecting portions 32 (Figure 2) of a hinged door 33. The door 33 can swing on a hinge 34 between open positions, such as that shown in Figure 4, and a closed position (not shown) in which the door completely blocks the inlet 30. The connecting portions 32 are preferably resilient projections, such as those shown in Figure 2, that can snap-fit into respective apertures 31b in the perimeter wall 31a. However, the connection can be made in any convenient manner, such as, for example, by projections provided on the top cover that mate with apertures provided in the door 33.

Advantageously, the side 31a of the perimeter wall 31 may also be provided with one or more apertures (not shown) for receiving a corresponding number of locking clips that comprise a U-shaped frame 32a projecting in the same direction as the connecting portions 32. The U-shaped frames 32a support respective resilient flaps 32b that depend out of the plane of the frames. When the door is assembled to the top cover 24, the flaps 32b can bend up into the plane of the frames 32a to allow insertion into the respective apertures therefor. Once through the apertures, the flaps 32b can flex back and grip behind the wall in which the apertures are formed and thus prevent the door 33 from being removed from the top cover 24. It will be appreciated that when these locking clips are provided, the number of connecting portions 32 may be reduced. It is envisaged that in some cases, the connecting portion 32 might be entirely replaced by locking clips 32a, 32b.

The side 31 c of the perimeter wall 31 opposite the side 31a is provided with means for snap-fit engaging co-operating means provided on the side of the door 33 opposite to the hinged side. These snap-fitting means enable the door to be locked in the closed position once the container portion 12 is full, so that the waste it contains cannot escape. Preferably, these snap-fitting means are arranged such that once the door is locked in its closed position, it cannot be re-opened. In the illustrated embodiment, the snap-fitting means comprises two lugs, or flaps 35 that can snap-fit into apertures (not shown) provided in the perimeter wall 31. However, this is not to be taken as limiting and any suitable means of which many will be readily apparent to the skilled person, may be used.

The door 33 may be recessed in the manner of a tray as shown. By recessing the door in this way, the door can serve as a tapered plug to better prevent sharps from poking out from the inlet or leakage of fluids in the event the waste disposal container 10 is dropped or knocked over after the door has been closed. In the case of a recessed door 33, it is advantageous to provide pads 36 to facilitate the pressing of the door in order to snap-fit the lugs 35 into the apertures in the side 31c of the perimeter wall 31. It may also be advantageous to provide the top cover with projections (not shown) adjacent the apertures for the lugs 35 to prevent accidental locking of the door 33. It will be appreciated that these need only be small projections that provide a small resistance to closure and past which the door can be pushed to reach its locked position with a relatively small deformation of the door.

Opposed downwardly extending vertical end walls 37 are provided at the shorter sides of the rectangular inlet 30. The end walls 37 are interconnected by downwardly extending vertical sidewalls 38 so as to define a generally rectangular passage 39 (best seen in Figure 6). The passage 39 leads downwardly from the inlet 30 towards the container portion opening 22. Respective inclined walls 40 are provided inwardly of the sidewalls 38. The inclined walls 40 extend parallel to the sidewalls 38 and project downwardly from the inlet 30 to a lesser extent than the sidewalls 38. The walls 40 define a restriction in the passage 39 at its upper end immediately downstream of the inlet 30.

A pivot bearing 46 (Figure 6) is provided on, or adjacent to, each end wall 37. The pivot bearings 46 provide support for a tumbler device 50 (Figures 3, 4 and 7) that is arranged for controlling access to the container portion 12. The tumbler device 50 is provided with respective spindles 52 (Figure 7) that snap-fit into the pivot bearings 46 so that it can rotate about a pivot axis defined by the bearings 46 and running parallel to the walls 38, 40. As illustrated by Figures 3 and 4, the tumbler device 50 can pivot between a blocking position (Figure 3) in which the inlet 30 and therefore access to the interior of the medical waste container 10 is blocked and a non-blocking position (Figure 4) in which waste can be deposited on a ledge 54 defined by the tumbler device 50.

Referring primarily to Figures 3, 4 and 7, the tumbler device 50 comprises an arcuate back wall 56 and a planar wall 58 that connects the back wall 56 to a further wall that defines the ledge 54. The wall defining the ledge 54 and the planar wall 58 are mutually inclined to define an obtuse angle therebetween. The free end of the arcuate back wall 56 is provided with a counterbalancing mass in the form of a series of ribs 60 positioned across the entire width of the back wall. At the free end of the ledge 54, there is a counterbalancing mass comprising a lip 62 on the inner side of which there are counterbalancing ribs 64 and a wall 66 that extends generally parallel to the lip and interconnects the ends of the ribs 64. In the embodiment, the counterbalancing masses are equal.

The counterbalancing masses are selected to bias the tumbler device 50 such that it will normally sit in the non-blocking position shown in Figure 4. With the tumbler device in this position, if an item of medical waste, such as a hypodermic syringe is dropped through the inlet 30, it will land on the ledge 54. In most cases, the weight of the syringe will be sufficient to cause the tumbler device to rotate anti-clockwise (as viewed in Figures 3 and 4) towards the position shown in Figure 3, allowing the syringe to drop through the opening 22 into the container portion 12. As shown in Figure 3, as the ledge 54 swings downwardly, the arcuate back wall 56 rotates upwardly to a position in which it blocks the narrow end of the passage 39 adjacent the inlet 30. Accordingly, while access to the container portion 12 is permitted for the waste held on the ledge 54, it is not possible for someone to pass their hand into the interior of the container portion. This is of course important as the waste, which may include sharps, such as the needles of hypodermic syringes, or perhaps scalpel blades, could be contaminated.

Due to the counterbalancing masses, once the waste has dropped from the ledge 54, the tumbler device 50 will normally automatically rotate back to the non-blocking position, shown in Figure 4. In this position, it is still not possible for someone to insert his or her hand into the container portion 12. As can be seen, the geometry of the planar wall 58 and adjacent inclined wall 40 are such that a substantially continuous blocking wall is provided to the right of the inlet 30 (as viewed in Figure 4). Opposite that blocking wall, the geometry of the ledge 54 and adjacent side and inclined walls 38, 40 are such that a tortuous path is defined. Although the lip 62 does not reach the peripheral wall 18 so that a relatively small gap 68 is left between them, the tortuous nature of the path from the inlet 30 to the gap 68 is such that it is not possible for a human hand to reach through to the gap 68 except, perhaps, by considerable exertion involving the deformation of parts of the waste disposal container.

If desired, the ledge 54 and/or lip 62 could be extended to substantially close the gap 68. However, it is preferred that a gap 68 is provided. The gap 68 provides the advantage that if the weight of an item of waste thrown into the inlet 30 is insufficient to cause the tumbler device 50 to rotate (this is likely to be the case if a syringe needle is thrown in by itself), the item can slide down the ledge 54 and through the gap 68 into the container portion 12. Thus the danger of having waste left sitting on the ledge 54 is avoided.

A feature of the waste disposal container 10 is that it should not be possible to overfill it. This is because, the ledge 54 projects into the container portion 12 to a notional fill line 70. When the last deposited waste item brings the level of the waste in the container portion to the level of the fill line 70, the tumbler device 50 is prevented from returning from the blocking position shown in Figure 3 to the non-blocking position shown in Figure 4. Accordingly once filled, the narrow end of the passage 39 remains blocked and it is not possible to deposit further waste into the container portion 12. The radially outer side of the back wall 56 may bear a message, such as BIN FULL LOCK DOOR AND REPLACE, so that users are made fully aware that the container 10 is full and do not try to force the tumbler device back to the Figure 3 position. Once the container 10 is full, the door 34 can be snap-fitted into its closed position so that the waste is securely retained in the container portion 12, prior to disposal by incineration.

As shown in Figure 1, the waste disposal container unit 10 is provided with a carrying handle 80. The carrying handle 80 can be rotated between non-use positions such as that shown in Figure 1 and a carrying position in which it extends over the inlet 30. The carrying handle 80 is profiled to match the lengthways profile of the top cover 24 so that in the carrying position there is only a small gap between the underside of the handle and the top cover. The carrying handle 80 has a raised central portion 82 that allows a person's hand between the top cover and handle.

It is envisaged that the medical waste disposal container 10 will be used as a stand-alone unit. However, some users may prefer to secure the container 10 to a wall or the like. For this purpose, the container 10 may be supplied with a wall mounting bracket 100 and the container portion 12 configured to be secured to the wall-mounting bracket.

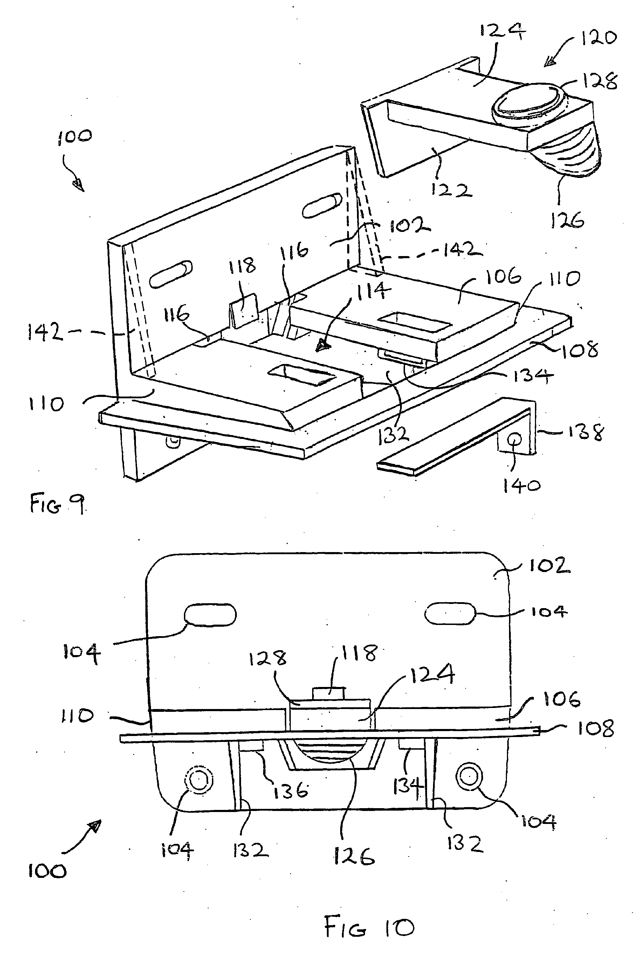

As shown in Figures 9 and 10, the wall mounting bracket 100 comprises a back plate 102 provided with a number of apertures 104 by means of which it can be secured to a wall or the like by means of screws or the like. A support plate 106 protrudes cantilever fashion from the back plate 102. The support plate 106 is surrounded by a U-shaped skirt 108. The support plate 106 has angled sides 110 so as to form a dovetail that can be received in a correspondingly shaped groove 112 provided in the bottom wall 16 of the container portion (see Figures 1 and 3). The depth of the support plate 106 is such that when it is received in the groove 112, adjacent areas of the bottom wall 16 are supported on the skirt 108.

The support plate 108 defines a slot 114 that extends from the back plate 102 to the skirt 108. At the end of the slot 114 adjacent the back plate, there are opposed recesses 116 that define a V-groove which extends at 90° to the plane of the slot 114. A resilient projection, or flap, 118 is provided on the back plate 102 adjacent the end of the slot 114. The slot and V-groove are configured to receive a spring clip 120. The spring clip 120 comprises a back wall 122 that has angled sides and is configured to fit in the V-groove defined by the recesses 116. A lever arm 124 projects from the back wall 122 such that when the spring clip is fitted in the slot 114, the upper surface of the arm is substantially flush with the upper surface of the support plate 106. The lever arm 124 is held in place in the slot 114 by the resilient flap 118. The free end of the lever arm 124 is provided with a thumb, or finger, grip 126 and a button 128 is provided on the lever arm upper surface adjacent the free end. The button 128 is configured to be received in a recess 130 provided in the bottom wall 16 within the groove 112.

Below the support plate 108, there are angled supporting webs 132, one to each side of the slot 114. Between the two supporting webs, there are opposed brackets 134, 136 arranged to receive a locking arm 138. The locking arm 138 has a through-hole 140 for receiving the U-bolt of a padlock (not shown). The supporting webs 132 each have a through-hole 133 corresponding to the through-hole 140 and positioned such that when the locking arm 138 is received in the brackets 134, 136, the adjacent one will line up with the through-hole 140 so that the locking arm can be padlocked to the respective supporting web 132. It will be appreciated that it is not essential to provide a through-hole 133 in both webs 132, but doing so makes locking equally easy for left and right-handed users.

In use, a medical waste disposal container 10 can be fitted onto the bracket 100 by sliding the bottom wall 16 onto the support plate 108 while the lever arm 124 is levered downwards so that the support plate is received in the groove 112. When the container portion 12 has been pushed fully onto the support plate 108, the lever arm 124 is released so that it springs upwards and the button 128 is received in the recess 130. With the button 128 received in the recess 130, it is not possible to slide the container portion 12 along the support plate 108, and the dovetail configuration of the support plate and groove 112 makes it impossible to lift the container portion from the support plate. The medical waste disposal container is therefore securely held on the wall-mounting bracket 100 until such time as the lever arm 124 is depressed to release the button 128 from the recess 130.

For situations where greater security is required, the locking arm 138 is slotted into the brackets 134, 136 and a padlock snapped into place so that the locking arm cannot be removed. With the locking arm in place, it extends beneath the lever arm 124 so that the lever arm cannot be depressed. Therefore, until such time as the padlock is removed, the waste disposal container 10 is securely fixed to the wall-mounting bracket 100.

Advantageously ribs such as the rib 142 shown in Figure 9 can be provided so that there is a positive engagement between the tapered wall 18 and the wall mounting bracket 100 to provide a better indication of correct location of the container on the bracket.

The medical waste disposal container 10 will preferably be supplied to the end user with the container portion 12 and top cover 24 separate. This will allow the container portion 12 and cover portion 24 to be separately stacked in a container such as a cardboard box, allowing maximum packing efficiency. The end user would preferably receive a package comprising a plurality of nested container portions 12 and the same number of nested top covers 24. Of course, the nested container portions 12 and top covers 24 might for convenience be supplied separately in respective packages.

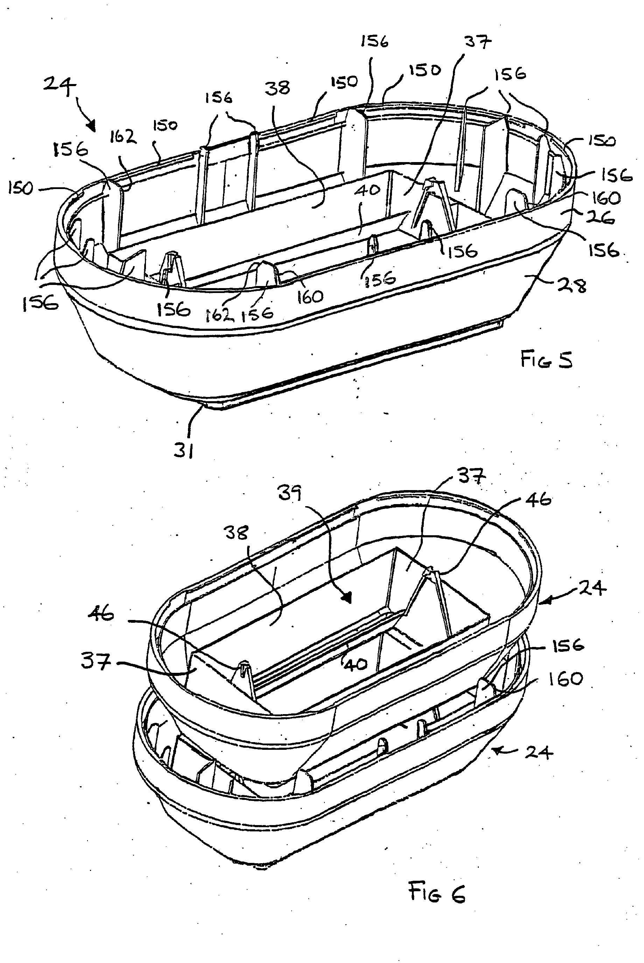

Referring to Figures 5, 6 and 8, the top cover 24 is provided with a lip 150, which is a second part of the snap-fitting engagement arrangement by which the top cover is securable to the container portion. The lip 150 is provided at the lower end of the first peripheral wall 26 on its inner surface. The lip 150 may be continuous or, as shown in the drawings, it may take the form of a series of discontinuous lip portions spaced around the peripheral wall 26 in such a way that each curved end and flat side portion of the wall 26 has an identical pair of lip portions.

To assist the end user in assembling the container portion 12 and top cover 24, a guide arrangement is provided to ensure that the two parts 20 and 150 of the snap-fitting engagement arrangement reliably and easily snap together with the top cover correctly and securely fitted to the container portion.

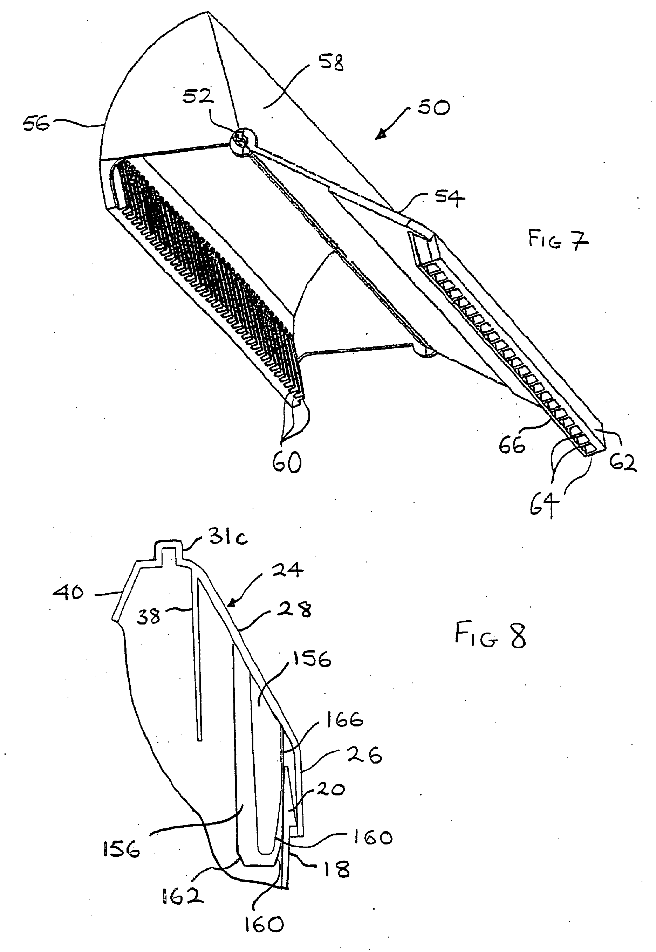

The guide arrangement comprises a plurality of rib-like guide members 156 provided on the inner surface of the top cover. In the embodiment, there are sixteen such guide members 156 spaced at intervals around the top cover 24. The guide members 156 extend downwardly (as viewed in Figures 3 and 4) from the inner surface of the second peripheral wall 28 of the top cover and end slightly below the lip 150. As best seen in Figures 3 and 4, there is a small gap or channel defined between the guide members 156 and the lip 150 through which the barb-like upper end 20 of the container portion 12 must pass in order to secure the container portion to the top cover 24. In the case of the guide members 156 positioned opposite spaces between the portions that make up the lip 150, this gap or channel 158 (Figure 8) is defined between the respective guide member 156 and the first peripheral wall 26. Each guide member 156 has an inclined face 160 that faces outwardly of the top cover 24. The inclined faces 160 are inclined upwardly and outwardly from the free ends of the guide members 156 so as to be inclined towards the channel 158 defined between the guide members 156 or first peripheral wall 26.

Referring to Figure 5, it will be seen that six of the guide members 156 are wider than the others. That is to say, these six guide members extend further inwards of the top cover than the remaining guide members. These six guide members are arranged and configured to provide support for another top cover 24 so that, as shown in Figure 6, the top covers can be nested together for packing and transportation. As can be seen in Figure 6, the uppermost of the two top covers sits in the lower top cover and is supported by an inclined face 162 of each of the wider 'supporting' guide members 156. The inclined faces 162 are arranged to engage the second peripheral wall 28 of the nested top cover and such that there are two support points on each of the relatively long straight sides of the peripheral wall 28 and one support point for each curved end portion of the peripheral wall 28. With this arrangement, the upper top cover 24 is positively located and supported in the lower top cover in a nested condition. It will be appreciated that the provision of the inclined faces 162 allows for a degree of self-alignment when the top covers are stacked so that if the upper top cover (as viewed in Figure 6) is slightly misplaced on initial placement in the lower top cover it will be guided automatically towards the correct position. This is of particular value if stacks of nested top covers 24 are to be assembled automatically, for example, by robots.

In use, when the end user comes to assemble a container portion 12 and top cover 24, the two parts will be brought together with the opening 22 facing the lower end of the top cover. As the two parts come together, any initial misalignment will be corrected as the inclined faces 160 of the guide members 156 engage the inner surface of the upper end 20 of the container portion 12. A sliding motion will be produced causing self-alignment of the two parts so that the top cover is correctly positioned over and covering the opening 22. Continual movement of the container portion 12 and top cover 24 then forces the barb-like upper end 20 of the container portion through the channels 58 to engage behind the lip 150 thereby snap-fitting the two parts together.

The guide members 156 positioned opposite portions of the lip 150 are configured such that when the barb-like upper end 20 of the container portion 12 has been pushed upwardly into the top cover 24 to a position in which it should engage behind the lip 150, the inner surface of the upper end 20 engages a face 166 of the respective guide member. The faces 166 face outwardly of the top cover 24 and extend upwardly from the upper end of the inclined faces 160 of the guide members 156. The faces 166 are configured such that if the upper end 20 of the container portion 12 is distorted slightly such that it would not otherwise properly snap-fit behind the lip 150, it will be engaged by a face(s) 166 in the affected area(s) and pushed, or deflected, firmly into the snap-fit position. It will be appreciated that the arrangement of the guide members 156 is such that they will have sufficient rigidity to perform this function and for practical purposes will not themselves deflect when engaging the container portion. Preferably, these guide members are essentially rigid members. By this arrangement a proper snap-fit engagement around the entire extent of the upper end 20 is reliably provided.

In tests with prototypes, it has been found that if the container portion 12 is placed on a flat surface and the top cover then placed over the opening 22, the top cover can be reliably and easily pushed down into its snap-fitted position by simply placing a hand over each end of the inlet 30 and pressing gently downwards. It has been found that this reliably results in a complete and proper security of the top cover on the container portion with little or no chance of improper fitting.

It will be understood that although there are sixteen guide members 156 in the embodiment, this should not be taken as limiting. There could be a greater or lesser number as required. Furthermore, in the embodiment, only six of the sixteen guide members 156 serve to support another top cover in nesting the top covers. Of course, all of the guide members 156 could be configured to provide a support function if desired. Equally a greater or smaller proportion of the guide members may provide the support function. As another, less preferred embodiment, there may be entirely separate members for providing the guide and support functions.

It will also be understood that the provision of the guide arrangement will prove advantageous even when the medical waste disposal container 10 is to be supplied the user pre-assembled. The ease with which the top cover 24 can be assembled to the container portion 24 should improve assembly times where hand assembly is employed and will facilitate automated assembly, such as by robots.

It will be appreciated that by making the wall mounting bracket in two parts, where the bracket is made as a plastics moulding, it is possible to produce the body part of the bracket from a cheaper plastics material, while producing the spring clip from a more expensive material that provides the necessary resilience, thereby making the production of the parts more economic. The body part could for example be made of polypropylene and the spring clip could be made of acetal, such as Delrin.

The top cover 24 and container portion 12 are preferably produced by injection moulding and suitable materials would be impact block copolymers or PP hetrophasic copolymers commonly known as IMP (Improved Mechanical Properties).

## Claims

1. A medical waste disposal container (10) comprising a hollow container portion (12) and a top cover (24) securable to an upper end region of said container portion by a snap-fitting engagement arrangement, said upper end region being provided with an opening (22) through which waste is received, said top cover including an inlet (30) through which waste can be inserted for passage to said opening and being provided with a first portion (150) of said snap-fitting engagement arrangement and said container portion being provided with a second portion (20) of said snap-fitting engagement arrangement which is arranged such that when said top cover and upper end region of the container portion are brought together, said first and second portions snap-fittingly engage to secure said top cover in a position covering said opening, said waste disposal container being further provided with a guide arrangement for guiding said top cover towards said position, said guide arrangement comprising guide members (156) provided on at least one of said top cover and container portion for engaging the other of said top cover and container portion as they are brought together for guiding said top cover to said position.

2. A medical waste disposal container as claimed in claim 1, wherein at least one said guide member (156) is arranged such that subsequent to said engagement of the other of said top cover (24) and container portion (12) it can deflect the respective said portion of the snap-fitting engagement arrangement to ensure that said first and second portions (20,150) snap-fittingly engage.

3. A medical waste disposal container as claimed in claim 2, wherein said at least one said guide members (56) is arranged to be relatively rigid such that it will not deflect when engaging the other of said top cover and container portion.

4. A medical waste disposal container as claimed in any one of the preceding claims, having at least two said guide members (156) provided on said top cover (24) and in the axial direction of the top cover projecting past the first portion (20) of the snap-fitting engagement portion such that when the top cover and container portion are brought together, said at least two guide members engage said container portion (12) prior to engagement of said first and second portions (20,150) of the snap-fitting arrangement.

5. A medical waste disposal container as claimed in any one of the preceding claims, having a plurality of said guide members (156) provided on said top cover (24) and arranged to provide support for a second such top cover (24) nested with said top cover.

6. A medical waste disposal container comprising a hollow container portion (12) having an opening (22) at an upper end region (20) thereof for receiving waste and a top cover (24) securable to said hollow container portion so as to cover said opening, said top cover including an inlet (30) through which waste can be inserted for passage to said opening and an internal support arrangement (156) for locating and supporting a second such top cover (24) in a nested condition.

7. A medical waste disposal container as claimed in claim 6, wherein said top cover is provided with a first portion (150) of a snap-fitting engagement arrangement and said container portion is provided with a second portion (20) of said snap-fitting arrangement, which is arranged such that when said top cover and an upper end region of the container portion are brought together, said first and second portions snap-fittingly engage to secure said top cover in position covering said opening.

8. A medical waste disposal container as claimed in any one of the preceding claims, wherein said first portion of said snap-fitting engagement arrangement comprises a lip (150) provided at a lower end region of said top cover (24) and at least some of said guide members (156) provided on said top cover extend opposite said lip so as to define respective channels (158) therebetween through which said second portion (20) of the snap-fitting engagement arrangement must pass prior to snap-fitting engagement with said lip.

9. A medical waste disposal container as claimed in claim 8, wherein said guide members (156) each have an inclined face (160) that is inclined towards the respective channel for aligning said top cover with the container portion and guiding said second portion (150) of the snap-fitting engagement arrangement towards said channel.

10. A medical waste disposal container as claimed in claim 8 or 9, wherein said lip (150) is provided at a lower end of said lower end region of said top cover (24) and comprises a plurality of discontinuous lip portions spaced apart around said lower end.

11. A medical waste disposal container as claimed in any one of the preceding claims, wherein said second portion (20) of said snap-fitting engagement arrangement comprises a barb defined by said upper end region of said container portion (12).

12. A medical waste disposal container as claimed in any one of the preceding claims, wherein said second portion (20) of the snap-fitting engagement arrangement extends continuously around said upper end region of said container portion (12).

13. A medical waste disposal container as claimed in any one of the preceding claims, wherein said top cover (24) comprises a first inclined continuous peripheral wall (26) and a second inclined continuous peripheral wall (28) extending upwardly from said first peripheral wall and being more inclined than said first peripheral wall.

14. A medical waste disposal container as claimed in any one of the preceding claims, wherein said top cover defines a passage (39) extending inwardly from said inlet (30) towards said opening (32) and is provided with a tumbler device (50), said tumbler device being rotatable from a first position in which a first portion (54,58) thereof substantially blocks access from said passage to said opening while an end of said passage adjacent said inlet is unblocked and a second position in which a second portion (56) thereof blocks said end of the passage and said opening is substantially unblocked to permit waste to pass into said container portion.

15. A medical waste disposal container as claimed in claim 14, wherein said tumbler device (50) is counterbalanced so as to be biased to said first position.

16. A medical waste disposal container as claimed in claim 15, wherein said first portion of the tumbler device comprises a ledge (54) on which waste can be deposited and said second portion of the tumbler device comprises an arcuate wall (56), said arcuate wall having a free end provided with a counterbalancing mass comprising a plurality of ridges (60) and said ledge has a free end provided with a counterbalancing mass comprising a plurality of ridges (64).

17. A medical waste disposal container as claimed in claim 16, wherein said counterbalancing masses are substantially equal.

18. A medical waste disposal container as claimed in any one of the preceding claims, wherein said container portion has two generally straight sides interconnected by opposed curved ends.

19. A package comprising a plurality of top covers as claimed in claim 5, or any one of the claims 8 to 18, when dependent on claim 5, or as claimed in claim 6 or 7, wherein said top covers (24) are in said nested condition.

20. A package as claimed in claim 19, further comprising a plurality of said container portions (12), said container being in a nested condition.

21. A wall mounting bracket (100) for a medical waste disposal container, said bracket comprising a body portion (102,106) that defines a support area for such a medical waste disposal container and a separate resilient locking device (120) carried by said body portion and having a normal position in which it engages a said container mounted to said body portion to lock said container to the body portion, said resilience permitting movement of said locking device from said locking position to permit said container to be mounted to or removed from said body portion.
